(19) **Europäisches Patentamt European Patent Office Office européen des brevets**

(11) **EP 4 066 261 B1**

(12) **EUROPEAN PATENT SPECIFICATION**

(45) Date of publication and mention of the grant of the patent:
**06.05.2026 Bulletin 2026/19**

(21) Application number: **20824645.4**

(22) Date of filing: **26.11.2020**

(51) International Patent Classification (IPC):
*G16H 30/40* *(2018.01)*

(52) Cooperative Patent Classification (CPC):
**G16H 30/40**

(86) International application number:
**PCT/IB2020/061175**

(87) International publication number:
**WO 2021/105918 (03.06.2021 Gazette 2021/22)**

(54) **METHOD FOR ESTIMATING HEAT TRANSFER ENERGY PARAMETERS IN AN ENCEPHALON**

VERFAHREN ZUR SCHÄTZUNG VON WÄRMEÜBERTRAGUNGSENERGIEPARAMETERN IN EINEM ENZEPHALON

MÉTHODE D'ESTIMATION DE PARAMÈTRES D'ÉNERGIE DE TRANSFERT DE CHALEUR DANS UN ENCÉPHALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2019 IT 201900022419**

(43) Date of publication of application:
**05.10.2022 Bulletin 2022/40**

(73) Proprietor: **Fondazione IRCCS Ca' Granda - Ospedale Maggiore Policlinico 20122 Milano (IT)**

(72) Inventors:
• **RANGO, Mario**
 **20139 Milano (IT)**
• **SQUARCINA, Letizia**
 **35125 Padova (IT)**

(74) Representative: **Bird & Bird Società tra Avvocati S.r.l.**
 **Via Porlezza, 12**
 **20123 Milano (IT)**

(56) References cited:
**WO-A1-2013/098690      US-A1- 2004 242 976**

• **HUAN WANG ET AL: "Brain temperature and its fundamental properties: a review for clinical neuroscientists", FRONTIERS IN NEUROSCIENCE, vol. 8, 8 October 2014 (2014-10-08), XP055714442, DOI: 10.3389/ fnins.2014.00307**

# EP 4 066 261 B1

**Description**

FIELD OF THE INVENTION

The present invention relates to the field of the acquisition and processing of data for diagnostic purposes.

In more detail, the present invention relates to a method for estimating energy parameters related to the heat transfer in an encephalon, in particular a non-invasive method and useful for diagnostic purposes for estimating the heat flow within the encephalon.

STATE OF THE ART

It is known that the temperature of the encephalon is not uniform and that there are heat flows inside the encephalon. The article by Huan Wang et al. entitled "Brain temperature and its fundamental properties: a review for clinical neuroscientists" (in Frontiers in Neuroscience, vol. 8, 8 October 2014) deals with these issues, but does not quantify or propose any calculations related to such heat flows.

Different therapeutic methods envisage the monitoring of the internal temperature of the encephalon during various types of therapy, e.g. during thermal ablation for curing cancer.

Currently, the monitoring of the internal temperature of the human body is prevalently performed invasively. In some circumstances such as, for example, the measurement of the temperature of the encephalon, the insertion of probes or other types of devices is considered to be too dangerous or risky.

Magnetic resonance techniques have recently been developed that can estimate the temperature of internal organs such as the encephalon. Such techniques do not provide any indications regarding heat exchanges between areas internal to the encephalon

ABSTRACT

The general object of the present invention is to improve the prior art from one or more points of view.

The invention is defined by the appended claims that form an integral part of the present description.

Specifically, the estimate of conductive flows in the encephalon is performed through discretization and numerical calculation; more precisely, the thermal conductivity and temperature distribution in the encephalon is discretized and then a calculation on the finite volumes of the "general heat conduction equation" is performed.

LIST OF FIGURES

The present invention shall become more readily apparent from the detailed description that follows to be considered together with the accompanying drawings in which:

Fig. 1 shows a schematic representation of a method according to the present description.

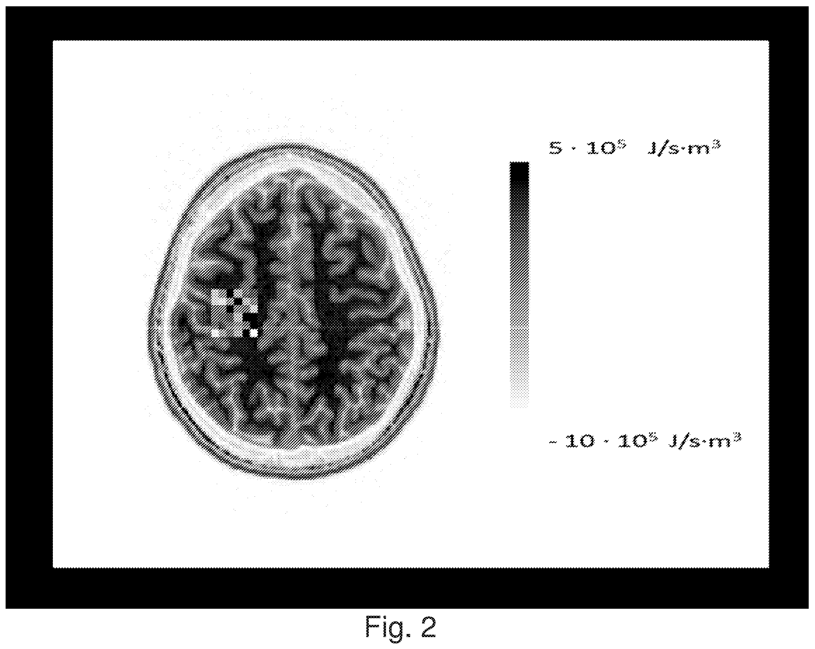

Fig. 2 shows a distribution of conductive heat flows in a portion of encephalon that can be obtained through the performance of a method according to the present description.

As can be easily understood, there are various ways of practically implementing the present invention which is defined in its main advantageous aspects in the appended claims and is not limited either to the following detailed description or to the appended claims.

DETAILED DESCRIPTION

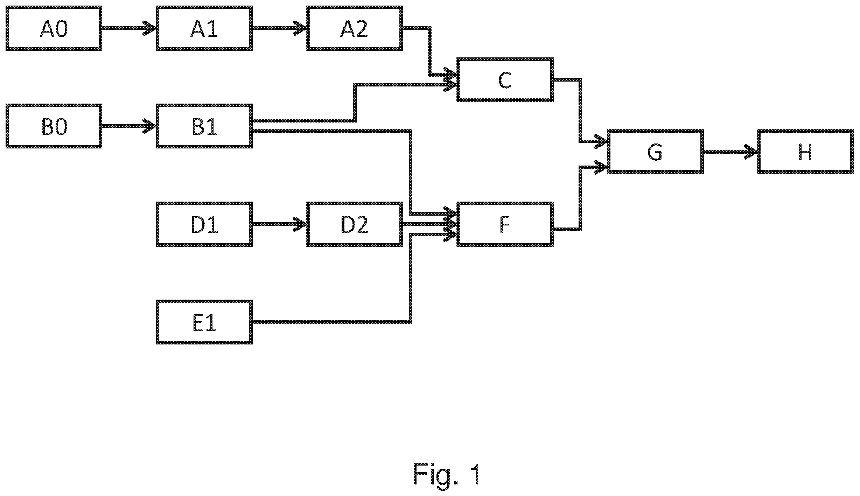

The subject matter of the present invention is a method for estimating heat transfer energy parameters in an encephalon, represented visually by way of example in Fig. 1.

In general, the method comprises the steps of:

A1) acquiring composition data regarding matter distribution in the encephalon;
B1) acquiring cerebral temperature data regarding temperature distribution in the encephalon;
C1) calculating a thermal conductivity distribution in the encephalon as a function of on the composition data;
C) calculating a distribution of conductive heat flows in the encephalon as a function of the cerebral temperature data and on the thermal conductivity distribution using Fourier's heat conduction equation.

In this way, a quantitative estimate of the conductive heat flows is obtained.

2

Typically, in step A1, the composition data are discretized into volumetric units.

Typically, in step B1, the temperature data are discretized into volumetric units.

Typically, in step A2, the thermal conductivity distribution is discretized into volumetric units.

As will become clearer below, for technological reasons, the composition and temperature could be discretized into volumetric units of different sizes, still referring to the same portion of encephalon and therefore we talk about "first volumetric units" and "second volumetric units".

Typically, the distribution of conductive heat flows is calculated through a finite volume calculation according to Fourier's heat conduction equation. In practice, the encephalon (or a portion of the encephalon) is split into small rectangular parallelepiped shaped volumes (the cube is a special case); if a small volume is considered, the equation is used to calculate the heat that flows from this small volume towards each of the six small volumes adjacent to its six faces; obviously, the sign of the value of the heat flow corresponds to the direction of the heat flow.

Advantageously, in step A1 a distribution of white matter and/or grey matter and/or cerebrospinal fluid is associated with respective first volumetric units.

Advantageously, in step B1 a cerebral temperature value is associated with each second volumetric unit, in particular the cerebral temperature value is assigned to a central point of the respective second volumetric unit.

Advantageously, in step A2, quantities of white and/or grey matter and/or cerebrospinal fluid contained in said second volumetric unit are calculated for each second volumetric unit.

Advantageously, the distribution of conductive heat flows calculated in step C is discretized into second volumetric units.

Each second volumetric unit comprises a certain number of first volumetric units.

The method according to the present invention will be described below in more detail. The method comprises a composition data acquisition step A1 regarding a distribution of matter in the encephalon. In particular, such composition data correspond to a distribution of white and/or grey matter and/or cerebrospinal fluid distribution in the encephalon. Preferably, step A1 comprises the acquisition of structural magnetic resonance images or MRI images of the encephalon with a T1-weighted sequence, in particular through MP-RAGE sampling.

For example, the parameters used for magnetic resonance imaging are as follows:

repetition time or TR: 2300 ms;
echo time or TE: 2.88 ms;
inversion time or TI: 1100 ms;
flip angle: 12°;
slice thickness: 1 mm;
spatial resolution (isotropic): 1 mm x 1 mm x 1 mm;
matrix dimensions: 256 x 224 x 159.

Advantageously, such magnetic resonance imaging technique allows an anatomic representation of the encephalon with relatively high spatial resolution.

The method further comprises a step A0 of creating a first mesh (or calculation grid) representative of at least a part of the encephalon, in which the encephalon (or a part thereof) is split into first volumetric units. In particular, the first volumetric units correspond to voxel of the aforesaid structural magnetic resonance imaging.

The composition data deriving from the structural magnetic resonance images are processed for generating a distribution of white and/or grey matter and/or cerebrospinal fluid in the encephalon discretized on the first mesh. In particular, this process is performed through the FSL FAST function, following which each first volumetric unit is classified as white material, or grey material or cerebrospinal fluid.

The method further comprises a cerebral temperature data acquisition step B1. Such data relate to a temperature distribution in the encephalon and are preferably acquired through magnetic resonance spectroscopy or MRS. Such acquisition can be performed according to the technique known as CSI3D and can have parameters equal or proximal to the following:

TR: 4000 ms;
TE: 270 ms;
spatial resolution: 4 mm x 4 mm x 8 mm;
matrix dimensions: 16 x 16 x 8;
No withdrawal of water.

Advantageously, such parameters allow a relatively accurate estimate of the temperature of the encephalon and reduce calculation errors.

The method further comprises a step B0 of creating a second mesh representative of the encephalon, in which the same encephalon is split into second volumetric units that are larger than the volumetric units of the first mesh and contain a plurality of first volumetric units. In particular, the second volumetric units correspond to voxel of the aforesaid magnetic resonance spectroscopy, which are larger than the voxel of the structural magnetic resonance images because of the lower resolution of spectroscopy with respect to structural magnetic resonance.

The cerebral temperature data deriving from the magnetic resonance spectroscopy are processed to generate a discretized temperature distribution in the encephalon on the second mesh so that each second volumetric unit is associated with a cerebral temperature value. In particular, the cerebral temperature value is assigned to a central point of the respective second volumetric unit.

The method further comprises a step A2 of calculating a thermal conductivity distribution in the encephalon as a function of the aforesaid composition data.

Preferably, the thermal conductivity distribution is calculated on the second mesh. In other words, each thermal conductivity value corresponds to an estimate of the conductivity of the matter contained in a second volumetric unit or of the matter interposed between two central points of respective second adjacent volumetric units, to which cerebral temperature values are assigned. In alternative embodiments a thermal diffusivity parameter can be calculated and used in the subsequent steps instead of the thermal conductivity.

Preferably, step A2 comprises the calculation, for each second volumetric unit, of a quantity of white and/or grey matter and/or cerebrospinal fluid contained in the second volumetric unit itself (or in equivalent units compatible with the calculation of heat exchanges between the second volumetric units of the second mesh), in particular of the fraction of white matter and/or grey matter and/or cerebrospinal fluid over the total volume. Such quantities of white and/or grey matter and/or cerebrospinal fluid are extrapolated from composition data associated with the first volumetric units contained in the second volumetric unit in question.

Subsequently, the step A2 comprises the calculation of a plurality of thermal conductivity values, each associated with a second distinct volumetric unit (or with equivalent units compatible with the second mesh) as a function of the respective quantity of white and/or grey matter and/or cerebrospinal fluid.

In a preferred embodiment, the thermal conductivity value of each second volumetric unit is estimated as a function of the quantity of white matter and grey matter contained therein. Furthermore, the quantity of cerebrospinal fluid previously determined for each second volumetric unit is used to exclude from the subsequent calculation steps the second volumetric units for which such quantity exceeds a certain predetermined threshold. Preferably, such predetermined threshold is 10% of the total volume of the second volumetric unit. In practice, such expedient only affects a reduced number of second volumetric units, in particular the second volumetric units which are superposed with sacs of cerebrospinal fluid, whereas most of them only contain white and/or grey matter.

In the preferred embodiment, the thermal conductivity value is calculated as a linear combination of the thermal conductivity values of the grey matter and of the white matter weighted according to a coefficient dependent on the respective quantities of matter within each second volumetric unit (in which the percentage of liquid is less than the predetermined threshold).

In particular, the following formula is used:

$$k_v = \mu_w k_w + \mu_g k_g$$

Wherein:

$k_v$ is the conductivity value of the second volumetric unit;
$\mu_w$ is the fraction of white matter in the second volumetric unit;
$k_w$ is the thermal conductivity of the white matter;
$\mu_g$ is the fraction of grey matter in the second volumetric unit;
$k_g$ is the thermal conductivity of the grey matter.

The method further comprises a step C of calculating a distribution of conductive heat flows in the encephalon as a function of the cerebral temperature data acquired in step B1 and the thermal conductivity distribution calculated in step A2.

Preferably, such calculation is performed on the second mesh and the aforesaid distribution of conductive heat flows comprises a conductive flow value for each second volumetric unit. Such value indicates the conductive heat flow of the second volumetric unit through the entire outer surface thereof. In other words, the conductive flow value calculated is representative of the conductive heat exchanges between the second volumetric unit in question and the adjacent second volumetric units (except for those neglected because of the quantity of cerebrospinal fluid).

Alternatively, for each second volumetric unit, the conductive flows between it and each of the adjacent second volumetric units can be calculated.

The conductive heat flow distribution is calculated through a finite volume calculation according to the "general heat conduction equation" which is expressed by "Fourier's law" according to which the quantity of heat is provided by the following formula:

$$Q = k\nabla^2 T$$

Wherein:

Q is the quantity of heat;
$k$ is the thermal conductivity;
$\nabla^2 T$ is the Laplacian of temperature with respect to space.

As mentioned, the "general heat conduction equation" is solved with the finite volume calculation, in particular said volumes are defined by the second mesh.

Furthermore, the "general heat conduction equation" can be approximated through the finite differences method and be solved for every surface $i$ of the second volumetric unit. For example, the equation can be approximated with the forward difference method:

$$Q_{v,i\ (i=1,n)} = k_v \nabla^2 T = k_v \frac{area_i}{distanza_{v,v+1}} \Delta T$$

Wherein:

$v$ represents the second volumetric unit being analysed;
$n$ is the total of the surfaces of the second volumetric unit;
$Q_{v,i}$ is the heat exchanged through the $i$-th surface of the second volumetric unit;
$k_v$ is the thermal conductivity of the second volumetric unit;
$area_i$ is the measurement of the extension of the surface $i$;
$distanza_{v,\ v+1}$ is the distance from the centre of the second volumetric unit v from the centre of the second adjacent volumetric unit $v+1$;
$\Delta T$ is the temperature difference between the second volumetric unit $v$ and the second adjacent volumetric unit $v+1$.

Alternatively and advantageously, the equation can be approximated with the central finite difference method.

Advantageously, the distribution of thermal conductivity values calculated in step A2 is used to determine the thermal conductivity value $k_v$ of the equation.

It is to be noted that the second volumetric units having a high concentration of cerebrospinal fluid, in particular a concentration greater than 10% of the total volume of the second volumetric unit, are excluded from the calculation.

In some embodiments, the method described can comprise additional steps in order to calculate further parameters in addition to the distribution of head conductive flows.

The method described can comprise a flow rate data acquisition step D1 relating to the blood flows in the encephalon. Preferably, step D1 comprises the acquisition of perfusion magnetic resonance images of the encephalon, in particular through the arterial spin labelling or ASL technique. Preferably, such data are acquired in the same acquisition step as step A1 and the data are collected on the first mesh. The data acquisition steps therefore consist of a single magnetic resonance session in which structural magnetic resonance, spectroscopy and perfusion magnetic resonance are performed in sequence.

Step D1 is followed by a step D2 of determining a distribution of blood flow rate values as a function of the flow rate data acquired. In particular, step D2 comprises interpolating the flow rate data on the second mesh so that each flow rate value determined in step D2 is representative of the blood flow rate crossing a respective second volumetric unit.

The method described can further comprise a blood temperature data acquisition step E1 relating to the temperature of the blood flows as above. Preferably, blood temperature data are acquired using a thermometer at body surfaces affected by haematic perfusion, e.g. the wrist, so as to detect the arterial temperature of the patient's body.

The temperature of the blood flows in the encephalon can be assumed to be equal to the arterial temperature measured. Alternatively, corrections can be performed to improve the estimate of the temperature of the blood flows in the encephalon, e.g. further temperature data can be collected in other areas of the body and the measured values combined. Alternatively, the measured temperature of certain organs or body regions can be used, as a surrogate for the blood temperature inside the encephalon. Such temperatures can be estimated in a non-invasive way through magnetic resonance spectroscopy techniques.

Subsequently, the method may comprise a step F of calculating a distribution of convective heat flows between the encephalon and the blood flows the cross it as a function of the aforesaid flow rate data acquired in step D1, the blood temperature data acquired in step E1 and the cerebral temperature data acquired in step B1.

Preferably, step F comprises the calculation of a convective heat flow value for each second volumetric unit (possibly except from those rejected due to the excessive cerebrospinal fluid content) as a function of the flow rate value and the cerebral temperature value associated therewith and the arterial temperature value.

In the preferred embodiment the convective heat flow is calculated through the following formula.

$$Q_B = \rho \, F \rho_B c_B (T - T_a)$$

Wherein:

$Q_B$ is the convective heat flow value extracted from each second volumetric unit from the blood flow;
$\rho$ is the density of the cerebral tissue;
$F$ is the value of the blood flow rate through the second volumetric unit;
$\rho_B$ is the density of the blood;
$c_B$ is the specific heat of the blood;
$T$ is the temperature of the second volumetric unit, acquired in step B1;
$T_a$ is the arterial temperature, acquired in step E1.

Subsequently, the method can comprise a step G of calculating a map of the metabolic heat generation of the encephalon, through an energy balance equation between the distribution of conductive heat flows calculated in step C, the distribution of convective heat flows calculated in step F and the same metabolic heat generation map.

In particular, the balance equation thus calculated assumes the stationariness of the total energy of the encephalon.

Preferably, step G comprises the calculation of a rate of metabolic heat produced for each second volumetric unit as a function of the conductive heat flow and convective heat flow values associated therewith.

$$Q_m = Q_b - Q_t$$

Wherein:

$Q_m$ is the rate of metabolic heat produced in the second volumetric unit;
$Q_b$ is the convective heat flow yielded to the blood flow by the second volumetric unit, calculated in step F;
$Q_t$ is the conductive heat flow entering into the second volumetric unit.

In particular, metabolic heat means the heat generated in the encephalon by glucose-oxygen combustion net of the heat removed for the separation of oxygen from haemoglobin.

Preferably, the method comprises a further step H of calculating a distribution of cerebral oxygen consumption rates as a function of the metabolic heat generation map, the reaction enthalpy between glucose and oxygen and the specific energy required for separation between oxygen and haemoglobin.

In particular, the calculation of step H is performed with the following formula:

$$\mathrm{rCMRO2} = \frac{Q_m}{(\Delta H^0 - \Delta H_b)\rho}$$

wherein:

rCMRO2 is the oxygen consumption rate in a second volumetric unit;
$Q_m$ is the metabolic heat rate produced in the second volumetric unit calculated in step G;
$\Delta H^0$ is the reaction enthalpy between glucose and oxygen;
$\Delta H_b$ is the energy required for the release of oxygen from the haemoglobin;
$\rho$ is the density of the cerebral tissue.

Further subject matter of the present invention is a medical apparatus configured for implementing the method described above, in particular for therapeutic or surgical uses. The described embodiments overcome the limitations of the prior art.

Advantageously, the method described provides useful parameters for the diagnosis and monitoring of patients.

## Claims

1. Medical apparatus adapted to implement a method for estimating heat transfer energy parameters in an encephalon through discretization and numerical calculation, the method comprising the steps of:

   A1) acquiring composition data regarding matter distribution in the encephalon, said composition data being discretized into volumetric units;

   B1) acquiring cerebral temperature data regarding a temperature distribution in the encephalon, said temperature data being discretized into volumetric units;

   A2) calculating a thermal conductivity distribution in the encephalon as a function of said composition data, said thermal conductivity distribution being discretized into volumetric units;

   C) calculating a distribution of conductive heat flows in the encephalon as a function of said cerebral temperature data and of said thermal conductivity distribution, said conductive heat flow distribution being calculated through a finite volume calculation of the "general heat conduction equation",

   wherein said step A1 comprising associating said composition data with respective first volumetric units,

   wherein said step A2 comprising calculating, for each second more extensive volumetric unit with respect to said first volumetric units, each second volumetric unit containing a plurality of first volumetric units, a quantity of white and/or grey matter and/or cerebrospinal fluid contained in said second volumetric unit, said quantity of white and/or grey matter and/or cerebrospinal fluid being extrapolated from the composition data associated with first volumetric units contained in said second volumetric unit, and

   wherein said step B1 comprises associating with each second volumetric unit a cerebral temperature value on the basis of said cerebral temperature data, in particular, the cerebral temperature value is assigned to a central point of the respective second volumetric unit.

2. Medical apparatus according to claim 1, wherein said composition data acquired correspond to a distribution of white matter and/or grey matter and/or cerebrospinal fluid.

3. Medical apparatus according to claim 1 or 2, wherein said step A1 comprises performing an acquisition of magnetic resonance images of the encephalon.

4. Medical apparatus according to one or more of the preceding claims, wherein said step B1 comprises acquiring magnetic resonance spectroscopy data of the encephalon.

5. Medical apparatus according to one or more of the preceding claims, further comprising the steps of:

   A0) creating a first mesh representative of at least a part of the encephalon in which said encephalon is split into first volumetric units;

   B0) creating a second mesh representative of at least a part of the encephalon in which said encephalon is split into second more extensive volumetric units with respect to said first volumetric units, each second volumetric unit containing a plurality of first volumetric units.

6. Medical apparatus according to claim 1, wherein said step A1 comprises performing an acquisition of magnetic resonance images of the encephalon and said first volumetric units correspond to voxel of said magnetic resonance images, and

   wherein said step B1 comprises acquiring magnetic resonance spectroscopy data of the encephalon and said second volumetric units correspond to voxel of said magnetic resonance spectroscopy.

7. Medical apparatus according to claim 1, wherein said thermal conductivity distribution comprises a plurality of thermal conductivity values, said step A2 comprising associating each thermal conductivity value with a second volumetric unit as a function of the respective quantity of white matter and/or grey matter and/or cerebrospinal fluid.

8. Medical apparatus according to claim 7, wherein each thermal conductivity value is calculated as a linear combination of the thermal conductivity values of the grey matter and of the white matter weighted according to a coefficient dependent on the respective quantities of matter.

9. Medical apparatus according to claim 7 or 8, wherein the calculation of the "general heat conduction equation" of said step C is performed on said second mesh using the thermal conductivity values of said thermal conductivity distribution.

10. Medical apparatus according to one or more of claims 5 to 9, said step C comprising overlooking the second volumetric units containing a quantity of cerebrospinal fluid greater than a predetermined threshold.

11. Medical apparatus according to one or more of the preceding claims, further comprising the steps of:

> D1) acquiring flow rate data related to blood flows in the encephalon;
> E1) acquiring blood temperature data related to the encephalon;
> F) calculating a distribution of convective heat flows between the encephalon and said blood flows as a function of said flow rate data, of said blood temperature data and of cerebral temperature data;
> G) calculating a map of metabolic heat generation of the encephalon by means of an energy balance equation between: said distribution of conductive heat flows, said distribution of convective heat flows and said map of metabolic heat generation.

12. Medical apparatus according to claim 11, further comprising the steps of:

> D2) determining a distribution of blood flow rate values as a function of said flow rate data, each flow rate value being representative of blood flow rate through a respective second volumetric unit;
> said step F comprising calculating a convective heat flow value for each second volumetric unit as a function of the blood flow rate value and the cerebral temperature value related to said second volumetric unit and the blood temperature data;
> said step G comprising calculating a rate of metabolic heat generation for each second volumetric unit as a function of the value of conductive heat flow and of the value of convective heat flow of said second volumetric unit.

13. Medical apparatus according to one or more of claims 10 to 12, further comprising the step of:
H) calculating a distribution of cerebral oxygen consumption rates as a function of at least said map of metabolic heat generation, of a reaction enthalpy between glucose and oxygen and of an energy required for separation between oxygen and haemoglobin.

**Patentansprüche**

1. Medizinische Vorrichtung, die ausgelegt ist, um ein Verfahren zur Schätzung von Wärmeübertragungsenergieparametern in einem Gehirn mittels Diskretisierung und numerischer Berechnung umzusetzen, wobei das Verfahren die folgenden Schritte umfasst:

> A1) Erfassen von Zusammensetzungsdaten bezüglich der Gewebeverteilung im Gehirn, wobei die Zusammensetzungsdaten in volumetrische Einheiten diskretisiert werden;
> B1) Erfassen von Hirntemperaturdaten bezüglich einer Temperaturverteilung im Gehirn, wobei die Temperaturdaten in volumetrische Einheiten diskretisiert werden;
> A2) Berechnen einer Wärmeleitfähigkeitsverteilung im Gehirn abhängig von den Zusammensetzungsdaten, wobei die Wärmeleitfähigkeitsverteilung in volumetrische Einheiten diskretisiert wird;
> C) Berechnen einer Verteilung von leitfähigen Wärmeströmen im Gehirn abhängig von den Hirntemperaturdaten und von der Wärmeleitfähigkeitsverteilung, wobei die Verteilung von leitfähigen Wärmeströmen mittels einer Finite-Volumen-Berechnung der "allgemeinen Wärmeleitungsgleichung" berechnet wird,
> wobei Schritt A1 das Assoziieren der Zusammensetzungsdaten mit jeweiligen ersten volumetrischen Einheiten umfasst,
> wobei Schritt A2 für jede zweite volumetrische Einheit, die in Bezug auf die ersten volumetrischen Einheiten umfangreicher ist, wobei jede zweite volumetrische Einheit eine Vielzahl von ersten volumetrischen Einheiten umfasst, das Berechnen einer Menge an weißer und/oder grauer Substanz und/oder Zerebrospinalflüssigkeit, die in der zweiten volumetrischen Einheit enthalten ist, umfasst, wobei die Menge an weißer und/oder grauer Substanz und/oder Zerebrospinalflüssigkeit aus den mit den ersten volumetrischen Einheiten, die in den zweiten volumetrischen Einheiten enthalten sind, assoziierten Zusammensetzungsdaten abgeleitet wird, und
> wobei Schritt B1 das Assoziieren eines Hirntemperaturwerts mit jeder zweiten volumetrischen Einheit auf der Grundlage der Hirntemperaturdaten umfasst, wobei der Hirntemperaturwert insbesondere einem zentralen Punkt der jeweiligen zweiten volumetrischen Einheit zugeordnet wird.

2. Medizinische Vorrichtung nach Anspruch 1, wobei die erfassten Zusammensetzungsdaten einer Verteilung von weißer Substanz und/oder grauer Substanz und/oder Zerebrospinalflüssigkeit entsprechen.

3. Medizinische Vorrichtung nach Anspruch 1 oder 2, wobei Schritt A1 das Durchführen einer Erfassung von Magnetresonanzbildern des Gehirns umfasst.

4. Medizinische Vorrichtung nach einem oder mehreren der vorhergehenden Ansprüche, wobei Schritt B1 das Erfassen von Magnetresonanzspektroskopiedaten des Gehirns umfasst.

5. Medizinische Vorrichtung nach einem oder mehreren der vorhergehenden Ansprüche, ferner umfassend die folgenden Schritte:

A0) Erstellen eines ersten Netzes, das zumindest für einen Teil des Gehirns repräsentativ ist, in dem das Gehirn in erste volumetrische Einheiten unterteilt ist;
B0) Erstellen eines zweiten Netzes, das zumindest für einen Teil des Gehirns repräsentativ ist, in dem das Gehirn in zweite volumetrische Einheiten unterteilt wird, die in Bezug auf die ersten volumetrischen Einheiten umfangreicher sind, wobei jede zweite volumetrische Einheit eine Vielzahl von ersten volumetrischen Einheiten enthält.

6. Medizinische Vorrichtung nach Anspruch 1, wobei Schritt A1 das Durchführen einer Erfassung von Magnetresonanzbildern des Gehirns umfasst und die ersten volumetrischen Einheiten Voxeln der Magnetresonanzbilder entsprechen, und
wobei Schritt B1 das Erfassen von Magnetresonanzspektroskopiedaten des Gehirns umfasst und die zweiten volumetrischen Einheiten Voxeln der Magnetresonanzspektroskopie entsprechen.

7. Medizinische Vorrichtung nach Anspruch 1, wobei die Wärmeleitfähigkeitsverteilung eine Vielzahl von Wärmeleitfähigkeitswerten umfasst, wobei Schritt A2 das Assoziieren jedes Wärmeleitfähigkeitswerts mit einer zweiten volumetrischen Einheit abhängig von der jeweiligen Menge an weißer Substanz und/oder grauer Substanz und/oder Zerebrospinalflüssigkeit umfasst.

8. Medizinische Vorrichtung nach Anspruch 7, wobei jeder Wärmeleitfähigkeitswert als lineare Kombination der Wärmeleitfähigkeitswerte der grauen Substanz und der weißen Substanz, gewichtet nach einem von den jeweiligen Substanzmengen abhängigen Koeffizienten, berechnet wird.

9. Medizinische Vorrichtung nach Anspruch 7 oder 8, wobei das Berechnen der "allgemeinen Wärmeleitungsgleichung" aus Schritt C auf dem zweiten Netz unter Verwendung der Wärmeleitfähigkeitswerte der Wärmeleitfähigkeitsverteilung durchgeführt wird.

10. Medizinische Vorrichtung nach einem oder mehreren der Ansprüche 5 bis 9, wobei Schritt C das Übersehen der zweiten volumetrischen Einheiten, die eine Menge an Zerebrospinalflüssigkeit enthalten, die größer als ein vorbestimmter Schwellenwert ist, umfasst.

11. Medizinische Vorrichtung nach einem oder mehreren der vorhergehenden Ansprüche, ferner umfassend die folgenden Schritte:

D1) Erfassen von Durchflussraten im Zusammenhang mit Blutflüssen im Gehirn;
E1) Erfassen von Bluttemperaturdaten im Zusammenhang mit dem Gehirn;
F) Berechnen einer Verteilung konvektiver Wärmeströme zwischen dem Gehirn und den Blutflüssen abhängig von den Durchflussratendaten, den Bluttemperaturdaten und den Hirntemperaturdaten;
G) Berechnen einer Karte der metabolischen Wärmeerzeugung des Gehirns mittels einer Energiebilanzgleichung zwischen der Verteilung leitfähiger Wärmeströme, der Verteilung konvektiver Wärmeströme und der Karte der metabolischen Wärmeerzeugung.

12. Medizinische Vorrichtung nach Anspruch 11, ferner umfassend die folgenden Schritte:

D2) Bestimmen einer Verteilung von Blutdurchflussratenwerten abhängig von den Durchflussratendaten, wobei jeder Durchflussratenwert repräsentativ für die Blutdurchflussrate durch eine jeweilige zweite volumetrische Einheit ist,

wobei Schritt F das Berechnen eines konvektiven Wärmestromwerts für jede zweite volumetrische Einheit abhängig von dem Blutdurchflussratenwert und dem Hirntemperaturwert im Zusammenhang mit der zweiten volumetrischen Einheit und den Bluttemperaturdaten umfasst,

wobei Schritt G das Berechnen einer Rate der metabolischen Wärmeerzeugung für jede zweite volumetrische Einheit abhängig von dem Wert des leitfähigen Wärmestroms und dem Wert des konvektiven Wärmestroms der zweiten volumetrischen Einheit umfasst.

**13.** Medizinische Vorrichtung nach einem oder mehreren der Ansprüche 10 bis 12, ferner umfassend den folgenden Schritt:

H) Berechnen einer Verteilung zerebraler Sauerstoffverbrauchsraten abhängig zumindest von der Karte der metabolischen Wärmeerzeugung, von einer Reaktionsenthalpie zwischen Glucose und Sauerstoff sowie von einer für die Trennung von Sauerstoff und Hämoglobin erforderlichen Energie.

**Revendications**

**1.** Appareil médical adapté à la mise en œuvre d'un procédé d'estimation des paramètres énergétiques de transfert de chaleur dans un encéphale par discrétisation et calcul numérique, le procédé comprenant les étapes suivantes :

A1) l'acquisition de données de composition concernant la distribution de la matière dans l'encéphale, lesdites données de composition étant discrétisées en unités volumétriques ;

B1) l'acquisition de données de température cérébrale concernant une distribution de température dans l'encéphale, lesdites données de température étant discrétisées en unités volumétriques ;

A2) le calcul d'une distribution de conductivité thermique dans l'encéphale en fonction desdites données de composition, ladite distribution de conductivité thermique étant discrétisée en unités volumétriques ;

C) le calcul d'une distribution des flux de chaleur conducteurs dans l'encéphale en fonction desdites données de température cérébrale et de ladite distribution de conductivité thermique, ladite distribution de flux de chaleur conducteurs étant calculée par le biais d'un calcul en volume fini de l'« équation générale de conduction de la chaleur »,

ladite étape A1 consistant à associer lesdites données de composition aux premières unités volumétriques respectives,

ladite étape A2 comprenant le calcul, pour chaque seconde unité volumétrique plus étendue par rapport auxdites premières unités volumétriques, chaque seconde unité volumétrique contenant une pluralité de premières unités volumétriques, d'une quantité de matière blanche et/ou grise et/ou de liquide céphalo-rachidien contenue dans ladite seconde unité volumétrique, ladite quantité de matière blanche et/ou grise et/ou de liquide céphalo-rachidien étant extrapolée à partir des données de composition associées aux premières unités volumétriques contenues dans ladite seconde unité volumétrique, et

ladite étape B1 consistant à associer à chaque seconde unité volumétrique une valeur de température cérébrale sur la base desdites données de température cérébrale, en particulier, la valeur de température cérébrale est attribuée à un point central de la seconde unité volumétrique respective.

**2.** Appareil médical selon la revendication 1, dans lequel lesdites données de composition acquises correspondent à une distribution de matière blanche et/ou de matière grise et/ou de liquide céphalo-rachidien.

**3.** Appareil médical selon la revendication 1 ou 2, dans lequel ladite étape A1 consiste à réaliser une acquisition d'images par résonance magnétique de l'encéphale.

**4.** Appareil médical selon l'une ou plusieurs des revendications précédentes, dans lequel ladite étape B1 comprend l'acquisition de données de spectroscopie par résonance magnétique de l'encéphale.

**5.** Appareil médical selon l'une ou plusieurs des revendications précédentes, comprenant en outre les étapes suivantes :

A0) la création d'une première maille représentative d'au moins une partie de l'encéphale dans laquelle ledit encéphale est divisé en premières unités volumétriques ;

B0) la création d'une seconde maille représentative d'au moins une partie de l'encéphale dans laquelle ledit encéphale est divisé en secondes unités volumétriques plus étendues par rapport auxdites premières unités volumétriques, chaque seconde unité volumétrique contenant une pluralité de premières unités volumétriques.

6. Appareil médical selon la revendication 1, dans lequel ladite étape A1 comprend la réalisation d'une acquisition d'images par résonance magnétique de l'encéphale et lesdites premières unités volumétriques correspondent à des voxels desdites images par résonance magnétique, et
ladite étape B1 comprenant l'acquisition de données de spectroscopie par résonance magnétique de l'encéphale et lesdites secondes unités volumétriques correspondent aux voxels de ladite spectroscopie par résonance magnétique.

7. Appareil médical selon la revendication 1, dans lequel ladite distribution de conductivité thermique comprend une pluralité de valeurs de conductivité thermique, ladite étape A2 consistant à associer chaque valeur de conductivité thermique à une seconde unité volumétrique en fonction de la quantité respective de matière blanche et/ou de matière grise et/ou de liquide céphalo-rachidien.

8. Appareil médical selon la revendication 7, dans lequel chaque valeur de conductivité thermique est calculée comme une combinaison linéaire des valeurs de conductivité thermique de la matière grise et de la matière blanche pondérées selon un coefficient dépendant des quantités respectives de matière.

9. Appareil médical selon la revendication 7 ou 8, dans lequel le calcul de l'« équation générale de conduction de la chaleur » de ladite étape C est effectué sur ladite seconde maille à l'aide des valeurs de conductivité thermique de ladite distribution de conductivité thermique.

10. Appareil médical selon l'une ou plusieurs des revendications 5 à 9, ladite étape C consistant à négliger les secondes unités volumétriques contenant une quantité de liquide céphalo-rachidien supérieure à un seuil prédéterminé.

11. Appareil médical selon l'une ou plusieurs des revendications précédentes, comprenant en outre les étapes suivantes :

   D1) l'acquisition de données relatives au débit sanguin dans l'encéphale ;
   E1) l'acquisition de données relatives à la température du sang dans l'encéphale ;
   F) le calcul d'une distribution des flux de chaleur convectifs entre l'encéphale et lesdits flux sanguins en fonction desdites données de débit, desdites données de température sanguine et des données de température cérébrale ;
   G) le calcul d'une carte de la production de chaleur métabolique de l'encéphale au moyen d'une équation d'équilibre énergétique entre : ladite distribution des flux de chaleur par conduction, ladite distribution des flux de chaleur par convection et ladite carte de la production de chaleur métabolique.

12. Appareil médical selon la revendication 11, comprenant en outre les étapes suivantes :

   D2) la détermination d'une distribution de valeurs de débit sanguin en fonction desdites données de débit, chaque valeur de débit étant représentative du débit sanguin à travers une seconde unité volumétrique respective ;
   ladite étape F comprenant le calcul d'une valeur de flux de chaleur convectif pour chaque seconde unité volumétrique en fonction de la valeur du débit sanguin et de la valeur de la température cérébrale relative à ladite seconde unité volumétrique et aux données de température du sang ;
   ladite étape G comprenant le calcul d'un taux de production de chaleur métabolique pour chaque seconde unité volumétrique en fonction de la valeur du flux de chaleur conductif et de la valeur du flux de chaleur convectif de ladite seconde unité volumétrique.

13. Appareil médical selon l'une ou plusieurs des revendications 10 à 12, comprenant en outre l'étape suivante :
H) le calcul d'une distribution des taux de consommation d'oxygène cérébral en fonction d'au moins ladite carte de génération de chaleur métabolique, d'une enthalpie de réaction entre le glucose et l'oxygène et d'une énergie nécessaire à la séparation entre l'oxygène et l'hémoglobine.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HUAN WANG et al.** Brain temperature and its fundamental properties: a review for clinical neuroscientists. *Frontiers in Neuroscience*, 08 October 2014, vol. 8